# EUROPEAN PATENT APPLICATION

(11) **EP 1 114 649 A2**
(43) Date of publication of application: **11.07.2001**
(21) Application number: 00670007.4
(22) Date of filing: 26.07.2000
(51) Int. Cl.: A61M 1/36, A61M 1/14

(54) **Anti-foam blood distributor**

(30) Priority: 26.07.1999 PT 10234099
(71) Applicant: PRONEFRO-Produtos Nefrologicos S.A., 4470 Maia (PT)
(72) Inventor: Colaço Gomes Covas, José Antonio, 4710 Braga (PT); Fernandes Gomes, Paulo Alexandre, 4700 Braga (PT)

(57) **Abstract**

The invention refers to a device (Figure 1) to be assembled in a chamber of a hemodialysis line to reduce the foam effect when blood entering the chamber shocks with the already accumulated blood. Less foam is created because the blood's average speed is reduced (obtained by a gradual increase of the area where the blood is conveyed), by diminishing the blood's impact against the chamber wall (by the design of the outlet tangent to the chamber's wall, at a height of (H), and by gradual direction transitions, ensured by radii (B), (C), (E) and (F). A blood curtain is created which flows slowly along about half of the chamber's wall, thus allowing visual inspection by treatment staff.

## Description

This invention refers to a device that eliminates foam during hemodialysis treatment when placed in a chamber of the hemodialysis line.

The device's shape causes a steady but significant slowdown in the blood's flow into the dialysis line chamber and also forms a blood curtain that flows smoothly along the chamber's internal wall.

Hemodialysis treatments compensate for the patient's renal deficiency. The process requires blood to flow through a circuit of tubes and various components with specific purposes. This circuit is called a hemodialysis line.

The chamber, one of its components, guarantees temporary accumulation of a minimum blood volume so that any air bubbles circulating in the hemodialysis line may be held back.

One of the chamber's problems is foam which forms when the blood entering the chamber shocks with the already accumulated blood. This problem is intensified during high blood flow that, in some cases, restricts the treatment time. The current method of overcoming that problem is to project the blood against the chamber's wall without reducing the speed. This solution, however, is not very effective.

This invention aims to solve this problem.

The features and advantages of this invention are outlined below and illustrated by the annexed drawings.

Figure 1 is a cross-section of the distributor's intermediate plane (symmetry line L-M in figure 2).

Figure 2 shows a layout of the distributor.

Figure 3 is a cross-section showing how the distributor is assembled in the chamber.

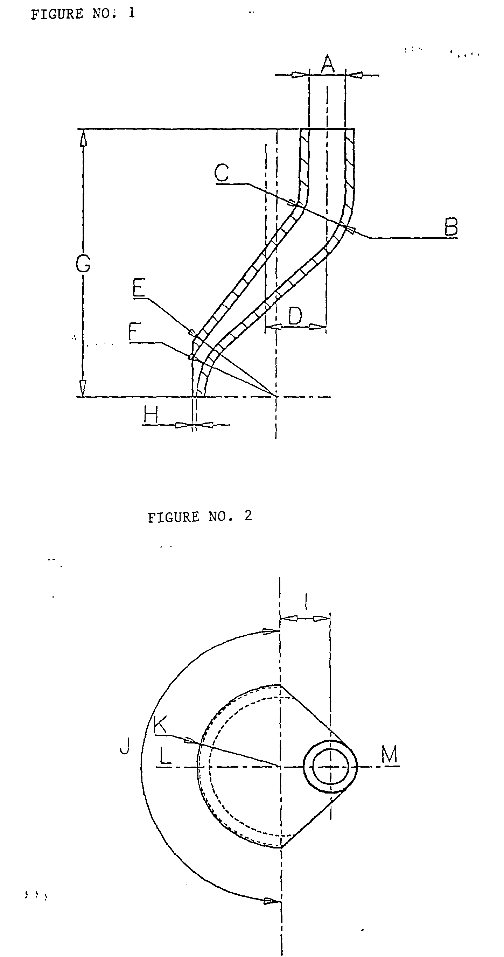

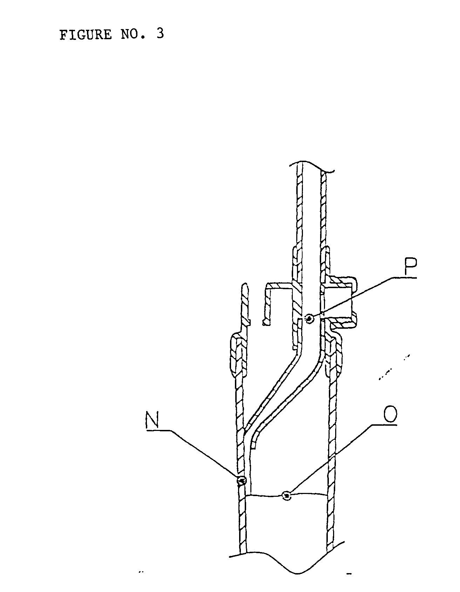

This invention allows the distributor to convey the blood from the chamber's intake (P in figure 3) until the internal wall of its body (N in figure 3) through a smooth route whose transversal area becomes progressively larger, producing a gradual reduction in blood speed. The part's first curve (shown by the radii of curves B and C in figure 1) is tangent to the intake and its subtle change of direction reduces the possibility of flow perturbation. The second curve (shown by the radii of curves E and F) is tangent to the 1^{st} curve and this curve tangent to the chamber's wall, thus dampening the transition of directions. As the semicircular shape of the distributor's outlet (indicated by radius K and arch J in figure 2) leaves a space until the chamber's inner wall (H in figure 1), a blood curtain is formed which flows downward at lower speed along the chamber's internal wall until the already accumulated blood level (O in figure 3). Therefore, the shock between the blood entering the chamber and the already accumulated blood takes place at low speed, thus reducing the formation of foam. The section's semicircular shape allows treatment staff to make a visual inspection, since the curtain occurs along only half the chamber's wall (angle J in figure 2).
To improve performance at low blood-flow speed, this invention has a triangular-teethed serrated edge on the lower rim along the outlet.

## Claims

1. Device to be assembled in the chamber of a hemodialysis line, conveying the blood flow from the intake (P) in the shape of the conductor tube until the chamber's wall (N), whose semicircular profile (indicated by radius K and arch J) adapt to the internal section of the chamber's body. It also has an intake curvature (indicated by radii B and C) tangent to the blood intake and an outlet curvature (indicated by the E and F radii) tangent to the chamber's wall.

2. Device in conformity with claim 1, characterised by an A intake diameter of about 4 mm and by an outlet section characterised by a curvature K of about 10 mm, by an arch J of about 180° and by a distance H of about 1 mm.

3. Device in conformity with any previous claims, characterised by a curvature of radius B and C of about 9 and 5 mm, respectively.

4. Device in conformity with any of the previous claims, characterised by two curvature of radii E and F of about 11 and 9 mm, respectively.

5. Device in conformity with any of the previous claims, characterised by a distance D of about 7 mm, by a distance G of about 32 mm, and by a distance I of about 6 mm.

6. Device in conformity with any of the previous claims, characterised by a triangular-teethed serrated edge on the bottom rim, and with a height of about 1 mm.
